# EUROPÄISCHE PATENTANMELDUNG

(11) **EP 1 782 817 A1**
(43) Veröffentlichungstag der Anmeldung: **09.05.2007**
(21) Anmeldenummer: 06123674.1
(22) Anmeldetag: 08.11.2006
(51) Int. Cl.: A61K 35/56

(54) **Pharmazeutischer Wirkstoff aus dem Mundsekret von Varanus, Timon, Lacerta und/oder Podarcis zur Behandlung von Tumorerkrankungen**

(30) Priorität: 08.11.2005 DE 102005053213; 10.11.2005 DE 102005053673; 16.11.2005 DE 102005054688
(71) Anmelder: TOXIMED GmbH, 80331 München (DE)
(72) Erfinder: Weickmann, Dirk, 80339, München (DE)
(74) Vertreter: Behnisch, Werner

(57) **Zusammenfassung**

Pharmazeutischer Wirkstoff zur Behandlung von Tumorerkrankungen, enthaltend in einer pharmazeutisch wirksamen Menge zumindest ungereinigtes Mundsekret, gewonnen von Tieren der Gattung Varanus, speziell der Arten kingorum, gilleni, glauerti, acanthurus, similis, storri, tristis, timorensis, der Gattung Timon, speziell der Art Timon lepida, Timon pater, der Gattung Lacerta, speziell der Art Lacerta viridis, der Art Lacerta trilineata und der Art Lacerta schreiberi, sowie der Gattung Podarcis, speziell der Art Podarcis muralis, Podarcis sicula, Podarcis melisellensis und Podarcis peloponnesiaca.

## Beschreibung

Die Nutzung biogener Gifte begann in der Geschichte der Menschheit schon in der Urzeit, als sie zur Erlegung von Beutetieren mit vergifteten Waffen diente. Zur gefahrlosen Anwendung dieser Gifte waren jedoch von Anfang an gewisse Grundkenntnisse über deren Behandlung und Wirksamkeit erforderlich. Die weiter durchgeführten Versuche, um die Zusammensetzung des chemischen Aufbaus biogener Gifte zu entschlüsseln, führten später zur gezielten Suche bestimmter Wirkstoffe als eigentliche Verursacher beobachteter Wirkungen.

Insbesondere die von Paracelsus (1493-1541) erhobene Forderung, die Wirkstoffe von Arzneipflanzen zu isolieren, und die zur Entwicklung der latrochemie, also der Chemie hinsichtlich ihres ärztlichen Anwendungsbereichs, beitrug, dürfte diese Bemühungen verstärkt haben. Vor allem die Kunst des Destillierens von Stoffen wurde in den Dienst der Forschung gestellt und lieferte eine Vielzahl ätherischer Öle und flüchtiger Stoffe. Aber für die Isolierung anderer Wirkstoffe oder gar für deren chemische Aufschlüsselung waren die damals bekannten Methoden unzureichend. Erst zu Beginn des 19. Jahrhunderts war die Entwicklung der technischen Fertigkeiten in der Chemie weit genug fortgeschritten, die Ära der Isolierung von reinen Wirkstoffen aus biologischem Material einzuleiten.

Zunächst nutzte man zur Abtrennung der gesuchten Wirkstoffe von den Begleitstoffen die Unterschiede in der Löslichkeit der untersuchten Substanzen in verschiedenen Lösungsmitteln. Beobachtet wurden hierbei, zum Beispiel mit Fällungsmitten, die Unterschiede im Verteilungsverhalten zwischen zwei nicht mischbaren, flüssigen Phasen, in der Flüchtigkeit und in der chemischen Reaktivität.

Einen gewaltigen Aufschwung in der Trenntechnik, dem Weg zur Ermittlung von Wirkstoffen zur Bekämpfung von Krankheiten, machte die Entwicklung chromatographischer Verfahren in der Mitte des 20. Jahrhunderts möglich. Ausgehend von der Verteilung zwischen einer mobilen und einer stationären flüssigen Phase, von der Adsorption, den Molekülsiebeffekten, dem lonenaustausch, der Affinität (insbesondere von Proteinen) zu bestimmten chemischen Verbindungen (z.B. Enzymsubstraten) und der Beweglichkeit geladener Moleküle im elektrischen Feld wurde eine Vielzahl neuer Trenntechniken entwickelt.

Derzeit werden Tumore, als die gefährlichsten und gefürchtetsten Krankheiten unserer Zeit auf eine sehr radikale und wenig umweltschonende Weise bekämpft. Als einfache, kennzeichnende Schlagworte können hier gelten:
Stahl, Strahl und Chemotherapie.
Das bedeutet einmal, dass Tumore, falls sie einigermaßen erreichbar, im Prinzip mit dem Stahl eines Messers herausgeschnitten, durch eine breitgefächerte Bestrahlung verbrannt oder über eine so genannte Chemotherapie mit auch gesunde Zellen angreifenden, aggressiven Zytostatika zerstört werden.

Sowohl bei normalen Behandlungen mit dem Skalpell als auch mit ionisierender Strahlung ist eine räumliche Begrenzung des Operationsgebiets nicht möglich. Es werden zwangsläufig auch gesunde Körperzellen vernichtet. Die unerwünschten Nebenwirkungen der Chemotherapie sind allgemein bekannt.

Im Gegensatz hierzu wurde aber auch versucht, eine Krebstherapie, die ihren Namen verdient, auf subtilere Weise zu ermöglichen. Zu diesem Zweck wurde auf den reichen Schatz der Natur zurückgegriffen. Es werden hierzu unter anderem viele aus giftigen Lebewesen isolierte, stark wirksame Stoffe in therapeutischen Dosen als Arzneistoffe genutzt.

So ist aus der DE 199 61 141 A1 ein pharmazeutischer Wirkstoff bekannt, bei dem gefunden wurde, dass Bestandteile der Spinnengifte von Spinnen der Familie Sicariidae zur Behandlung von Tumorerkrankungen verwendet werden können. Es werden hierbei in der Hauptsache ein Peptidtoxin aus dem Gift dieser Spinnenart, eine weitere aus dem Gift gewonnene, antagonistisch wirkende Substanz und/oder eine Kombination dieser Bestandteile medizinisch genutzt. Es kann dieser Wirkstoff zur Behandlung von Tumorerkrankungen sowie parallel bzw. unterstützend zu Tumoroperationen eingesetzt werden und Rest-Tumorgewebe zerstört werden. Bei der Therapie können genetisch veränderte Körperzellen (Tumorzellen) zerstört werden, da der betreffende Wirkstoff die veränderte Oberflächenstruktur solcher Zellen erkennt und komplikationsfrei abtötet. Der Gesamtgiftgehalt dieser Spinnenart, sozusagen ein Cocktail verschiedener Substanzen, ist auf Grund seiner bereits in geringen Dosen letalen Wirkung nicht pharmazeutisch einsetzbar.

In der Folgezeit wurden zahlreiche Wirkstoffe gegen verschiedenste Tumorarten und andere Krankheiten auf der Basis biogener Gifte und anderer biogener Wirkstoffe entwickelt. Für eine großtechnische Anwendung dieser Wirkstoffe werden jedoch große Mengen benötigt, die im Hinblick auf die geringen Mengen an Grundstoffen, die von den einzelnen Tierarten gewonnen werden können, nicht zu realisieren sind.

Es ist deshalb die Aufgabe der vorliegenden Erfindung, Mittel und Verfahren zur Behandlung von Tumorerkrankungen auf der Basis biogener Grundstoffe zur Verfügung zu stellen, die es erlauben, größere Mengen an Wirkstoff zu produzieren.

Diese Aufgabe wird von den Wirkstoffen mit den Merkmalen des Anspruchs 1 sowie den Verfahren der Ansprüche 7 und 8 gelöst.

Ein wesentlicher Aspekt bei der Gewinnung des erfindungsgemäßen Wirkstoffs ist der, dass das biogene Mundsekret von Waranen und Eidechsen gewonnen wird. Sowohl die Warane (Familie Varanidae) als auch die Eidechsen (Familie Lacertidae) sind in der Klasse der Klasse der Reptilien der Unterordnung der Schuppenechsen (Lacertilia) zugehörig. Die Warane entstammen dabei der Gattung Varanus, insbesondere von Tieren der Arten kingorum, gilleni, glauerti, acanthurus, similis, storri, tristis und timorensis. Die Eidechsen gehören zur Gattung Timon, insbesondere deren Arten lepida und pater, zur Gattung Lacerta, insbesondere deren Arten viridis, trilineata und schreiberi, und zur Gattung Podarcis, insbesondere deren Arten muralis, sicula, melisellensis und peloponnesiaca.

Das gewonnene Sekret wird in einer pharmazeutisch wirksamen Menge eingesetzt, d.h., das gewonnene Sekret wird in einer pharmazeutischen Zubereitung in einer Menge verwendet, die eine Wirkung in einem Patienten hervorruft. Dabei können die Sekrete der unterschiedlichen Tiere in einer pharmazeutischen Zubereitung allein oder in Kombination mehrer Sekrete unterschiedlicher Tiere enthalten sein.

Der pharmazeutische Wirkstoff kann zur Herstellung eines Medikaments verwendet werden. Das Medikament kann dabei der Behandlung unterschiedlicher Krebserkrankungen dienen und neben dem erfindungsgemäßen Wirkstoff weitere pharmazeutische Hilfsstoffe enthalten, die üblicherweise von einem Fachmann zur Herstellung von pharmazeutischen Zubereitungen verwendet werden.

Die Wirkstoffe der vorliegenden Erfindung werden vorzugsweise in Form einer solchen pharmazeutischen Zusammensetzung verwendet, in der sie mit geeigneten Trägern oder Trägerstoffen in Dosen vermischt werden, so daß die Erkrankung behandelt oder zumindest gelindert wird. Eine derartige Zusammensetzung kann (zusätzlich zu den Wirkstoffen und dem Träger) Verdünnungsmittel, Füllmaterialien, Salze, Puffer, Stabilisatoren, Solubilisierungsmittel und andere Materialien enthalten, die in der Technik wohlbekannt sind. Der Begriff "pharmazeutisch verträglich" ist als ein nichttoxisches Material definiert, das die Wirksamkeit der biologischen Aktivität des aktiven Inhaltsstoffes bzw. Wirkstoffes nicht stört. Die Auswahl des Trägers hängt vom Verabreichungsweg ab.

Die pharmazeutische Zusammensetzung kann zusätzlich weitere Mittel enthalten, die die Aktivität des Wirkstoffes steigern oder dessen Aktivität oder Verwendung bei der Behandlung ergänzen. Derartige zusätzliche Faktoren und/oder Mittel können in der pharmazeutischen Zusammensetzung enthalten sein, um eine synergistische Wirkung zu erzielen oder um Nebenwirkungen bzw. unerwünschte Wirkungen zu minimieren.

Techniken zur Formulierung bzw. Zubereitung und Verabreichung der Verbindungen der vorliegenden Anmeldung sind in "Remington's Pharmaceutical Sciences", Mack Publishing Co., Easton, PA, letzte Ausgabe, zu finden. Eine therapeutisch wirksame Dosis bezieht sich ferner auf eine Menge der Verbindung, die ausreicht, um eine Verbesserung der Symptome zu erreichen, beispielsweise eine Behandlung, Heilung, Prävention oder Verbesserung derartiger Zustände. Geeignete Verabreichungswege können beispielsweise orale, rektale, transmucosale oder intestinale Verabreichung und parenterale Verabreichung einschließen, einschließlich intramuskulärer, subkutaner, intramedulärer Injektionen ebenso wie intrathekaler, direkt intraventrikulärer, intravenöser, intraperitonealer oder intranasaler Injektionen. Die intravenöse Verabreichung an einen Patienten wird bevorzugt.

Eine typische Zusammensetzung für eine intravenöse Infusion kann so hergestellt werden, daß sie 250 ml sterile Ringer'sche Lösung und z.B. 10 mg pharmazeutischer Wirkstoff enthält. Siehe Remington's Pharmaceutical Science (15. Ausgabe, Mack Publishing Company, Easton, Ps., 1980).

Die oben genannten Arten sind im Folgenden näher beschrieben.

Warane bilden in der Unterordnung der Echsen (Sauria) eine eigene Familie (Varanidae). Es gibt insgesamt 32 verschiedene Arten von Waranen. Diese leben in den Tropen und Subtropen Afrikas, Asiens und Australiens. 17 Arten von ihnen kommen allein in Australien vor. Die kleinsten Arten werden nur ca. 20 cm lang. Die meisten von ihnen sind jedoch große, mächtige Tiere. Der größte von ihnen wird bis zu 3 Meter lang. Es ist der Komodo-Waran (Varanus komodoensis). Er kommt nur auf der Insel Komodo und auf wenigen umliegenden Inseln des südostasiatischen Sund-Archipels vor.

Die Warane bilden eine, den Schlangen nahe stehende Familie. Sie haben einige Körpereigenarten, die den Schlangen sehr ähnlich sind. Eine ist zum Beispiel die lange, tief gespaltene Zunge. Mit der Zungenspitze können die Warane aus der Luft feinste Duftstoffe aufnehmen. Diese führen sie in das sogenannte Jacobsonsche Organ. Hierbei handelt es sich um ein spezielles, im Gaumen sitzendes Geruchsorgan. Dank ihres sehr gut ausgebildeten Geruchssinns können die Warane ohne Probleme den Spuren ihrer Beutetiere folgen. Der Körper der Warane ist lang und relativ breit und flach. Die Beine sind kräftig und an den Füßen sitzen starke gekrümmte Zehen. Der Schwanz ist lang und bei den größeren Vertretern sehr kräftig. Der Kopf sitzt auf einem deutlich erkennbaren Hals und ist flach, die Schnauze ist abgerundet. Bei vielen Arten wirkt die Haut etwas zu groß für den Körper, da sie am Hals und Rumpf Falten wirft. Die Schuppen der Warane sind sehr unterschiedlich gefärbt. Die Farbpalette reicht von Grau über gedecktes Grün bis hin zu Braun. Manche Arten weisen eine stark ausgeprägte Musterung, wie Querstreifen und Punkte, auf.

Die Fortbewegungsmöglichkeiten der Warane sind äußerst vielseitig. Alle Warane sind gute Schwimmer und auch Taucher. Dabei legen sie die Beine nach hinten an den Körper und steuern mit ihrem langen Schwanz. Abgesehen von ausgewachsenen Komodowaranen sind alle Arten, von denen einige fast reine Baumbewohner sind, gute Kletterer. Trotz ihrer zum Teil massigen Gestalt sind Warane gute und schnelle Läufer. Die Warane sind vor allem Fleischfresser, jedoch haben sie auch eine Vorliebe für Eier. Sie können, genau wie die Schlangen, ihren Schlund besonders stark ausdehnen und dadurch auch sehr große Futterbrocken verschlingen. Sie jagen alle Tiere, die sie überwältigen können. Dies sind zum Beispiel Schlangen, Echsen, Kerbtiere, kleine Säugetiere, Vögel und sogar auch ganze Schildkröten. Warane verachten auch Aas nicht. Ein Komodowaran jagt sogar kleine Hirsche und Wildschweine.

Macht der Waran Jagd auf sehr große Beutetiere, dann hält er diese mit seinen krallenbesetzten Zehen fest und reißt mit den Zähnen große Fetzen los. Dies macht ihm meist keine Probleme, da seine Kiefer sehr stark sind. Obwohl das Fleisch und die Häute der Warane sehr geschätzt werden, ist dies nicht der Grund, warum die Bestände der Warane sich weltweit immer mehr verringern. Der eigentliche Grund dafür ist vielmehr das Fett, das man aus ihnen gewinnt. Dies wird vor allem zur Herstellung von Salben verwendet. Diese sollen gegen verschiedene Krankheiten helfen. Die Waransalbe ist besonders in China sehr begehrt.

Varanus kingorum wird auch Kings-Varan genannt und ist in Australien beheimatet. Varanus gilleni, oder auch Gillens Zwergwaran, lebt in Mittel-Australien. Varanus glauerti gehört zu den mittelgroßen Vertretern der Untergattung Odatria. Er ist ein Felsbewohner. Kopf, Körper und Gliedmassen zeigen eine schwärzliche oder dunkelbraune Färbung. Der Körper ist mit undeutlichen Querreihen großer heller Flecken bedeckt. Die Hinterbeine sind mit Querreihen kleiner Punkte gezeichnet. Ein deutlich sichtbarer, streng abgesetzter Temporalstreifen ist auf der Seite des Kopfes sichtbar. Der Schwanz ist mit abwechselnden hellen und dunklen Querbinden versehen. Bauch und Kehle sind weißlich. Die Kopfschuppen sind klein, unregelmäßig und glatt. Das Nasenloch steht seitlich, es befindet sich etwas näher zur Schnauzenspitze als zum Auge. 120 bis 160 Schuppenreihen sind um die Körpermitte angeordnet. Der Schwanz ist mehr oder weniger rund im Querschnitt, ohne Anzeichen eines Kiels auf der Oberseite. Er ist ungefähr doppelt so lang wie die Körperlänge. Die Schuppen sind an der Schwanzbasis glatt, zum Schwanzende gering gekielt. Die Gesamtlänge beträgt bis 80 cm. Die Verbreitung von Varanus glauerti erstreckt sich auf den äußersten Norden Australiens. Zwei nicht zusammenhängende Gebiete sind hier bekannt. In den Kimberleys findet man diesen Waran auf großen Felsbrocken, in deren Spalten er bei Gefahr verschwindet. Eine zweite Population lebt im Kakadu National Park, Northern Territory. Dort sind die Warane hauptsächlich an Bäumen anzutreffen. Ihre Nahrung besteht aus Insekten, Spinnen, Geckos und Skinken, die bei ihren Streifzügen durch die Felsspalten erbeuten.

Der Varanus acanthurus, oder auch Stachelschwanzwaran (Dornschwanzwaran) kann bis zu 70 cm Gesamtlänge erreichen. Er hat gelbe bis cremfarbene Augenflecken auf dem Rücken und dem Schwanz. Sie bewohnen große Teile der nördlichen Hälfte Australiens mit Ausnahme der Cape York Halbinsel. In der Natur bewohnt dieser Waran steinige Biotope, wo er auf Felsen sonnenbadet und stets auf der Hut vor Fressfeinden ist. Er ist ein höchst aktiver Jäger, der intensiv zwischen den Steinen nach Futter stöbert. Seine Nahrung besteht aus kleinen Vögeln, Eidechsen und Insekten.

Der Varanus similis wird auch als gefleckter Baumwaran bezeichnet. Der Varanus storri oder Zwergwaran erreicht eine Länge von bis zu 45 cm. Er ist hell- bis dunkelbraun und besitzt einen dicken bestachelten Schwanz. Der Trauerwaran oder Varanus tristis bewohnt fast ganz Australien, außer dem äußersten Süden und Südosten dieses Kontinents. Er erreicht eine Gesamtlänge von etwa 25 cm bis 30 cm. Er ernährt sich von Eidechsen und kleinen Vögeln.

Das Verbreitungsgebiet von Varanus timorensis erstreckt sich auf die Inseln Timor, Sawu und Semau in Süd-Indonesien. Dieser Waran bevorzugt ein weites Spektrum an Biotopen, meist trockene bis feuchte Wälder. Er bewohnt hauptsächlich Bäume und Palmen, aber auch Natursteinmauern. Varanus timorensis wird ca. 60 cm groß, wobei etwa 25 cm davon auf die Kopf-Rumpf-Länge entfallen. Diese Tiere haben meist eine dunkelgraue Grundfarbe. Über den Körper sind mehr oder weniger unregelmäßig weiße bzw. cremefarbene bis gelbe Flecke verteilt, die ein dunkles Zentrum haben und meist auch dunkel umrandet sind, so genannte Ozellen. Unterhalb des Auges verläuft ein heller Streifen, der sich hinter der Ohröffnung verliert. Mit zunehmendem Alter der Tiere wird dieser Streifen jedoch immer undeutlicher. Der Schwanz ist mehr oder weniger deutlich gebändert.Die Farbintensität der helleren Zeichnungselement des Körpers kann zwischen einzelnen Tieren beträchtlich variieren. Die Körperunterseite ist weiß bis gelblich, letzteres vor allem an Hals und Kehle, und mit dunklen Streifen, Ozellen, Flecken oder Punkten gemustert. Varanus timorensis kann 10 bis 15 Jahre alt werden.

Timon lepida, oft auch Timon lepidus genannt, ehemals Lacerta lepida, wird auch als Perleidechse bezeichnet. Sie ist die größte europäische Eidechse und kann eine Körperlänge von bis zu 80 cm erreichen, wobei 2/3 der Gesamtlänge der Schwanz einnimmt. Ihre Grundfarbe ist graugrün. Auf dem Rücken kommen grüne türkisfarbene und schwarze Schuppen vor. An den Flanken finden sich mehrere Reihen türkisfarbener Flecken. Die Unterseite ist gelblich gefärbt. Die Jungtiere sind gelbbraun bis grün mit hellen, schwarz umrandeten Augenflecken. Das Männchen verfügt über einen kräftigen Kopf mit einer aufgetriebenen Wangenregion. Der dicke Schwanz endet in einer lang gezogenen Spitze. Diese Eidechse ist ein guter Kletterer und ein schneller Läufer. Sie ist sehr scheu. Bei Bedrohung oder Revierstreitigkeiten wird der Rumpf vom Boden abgehoben und der Hals abgeflacht. Zwischen Männchen entstehen häufig Beißereien. Im Winter wird Winterruhe gehalten.

Das Vorkommen von Timon lepida erstreckt sich über die iberische Halbinsel, Süd- und Südwestfrankreich, Nordwestitalien und Nordwestafrika. Bevorzugter Lebensraum ist trockenes, locker bewachsenes Gelände mit Mauern und Steinhaufen. Die Perleidechse lebt in trockenem felsigem Gelände mit dornigen Büschen und braucht als tagaktives Tier viel Sonne. Zur Fortpflanzungszeit leben diese Eidechsen paarweise. Die Nahrung von Timon lepida besteht aus großen Insekten, Schnecken, Skorpionen, Würmern, Mäusen, kleinen Eidechsen, kleinen Schlangen, kleinen Vögeln und ab und zu auch süßen Früchten.

Timon pater wird auch als nordafrikanische Perleidechse bezeichnet und ist sehr selten.

Lacerta viridis oder Smaragdeidechse (östliche Smaragdeidechse) ist die mit Abstand größte heimische Eidechse. Sie erreicht eine Gesamtlänge von bis zu 40 cm. Am auffälligsten sind die fast einfarbig smaragdgrünen Männchen, deren Kehle sich zur Paarungszeit leuchtend blau färbt. Die Grundfärbung der Weibchen ist ebenfalls grün, allerdings mit starker, schwarzbrauner Sprenkelung. Manche Weibchen weisen auch zwei helle Längsstreifen auf. Die Jungtiere haben nach dem Schlüpfen einen einfarbig braunen oder kupferfarbenen Rücken. Halbwüchsige Tiere sind braun gemustert. Nach etwa zwei Jahren sind sie erwachsen und färben sich grün.

Als Lebensraum bevorzugt die Smaragdeidechse Böschungen in Südlage, welche bestimmte Strukturen aufweisen müssen: niedriges Gebüsch, offene Bodenstellen oder Felsen, insektenreiche Wiesen und lockeren Boden oder Sand. Erwachsene Männchen verteidigen ihr mehrere hundert Quadratmeter großes Revier eifersüchtig gegen andere Männchen. Die Weibchen legen ihre Eier je nach Witterung im Juni oder Juli an sonnigen Stellen mit lockerem Boden ab. Danach kümmern sich die Weibchen nicht mehr um das Gelege, die Sonnenwärme brütet die Eier aus. Nach zwei Monaten schlüpfen die Jungen. Erwachsene Tiere begeben sich im September in ihre Winterquartiere, die Jungen sind bis weit in den Oktober aktiv. Die Smaragdeidechse ernährt sich von allen Tieren, die sie überwältigen kann. Dies können neben Insekten und anderen Kleintieren auch andere Eidechsenarten und sogar eigene Jungtiere sein.

Lacerta trilineata oder die Riesensmaragdeidechse sieht im erwachsenen Zustand unseren einheimischen Smaragdeidechsen sehr ähnlich. Bei Freilandbeobachtungen ist eine Unterscheidung nur anhand der anders gefärbten Jungtiere möglich. Diese Eidechsen finden ihr Verbreitungsgebiet an Küstengebieten Jugoslawiens, in Griechenland, teilweise Bulgarien, Rumänien und der Türkei. Dort bewohnen sie Buschwerk und buschreiche Sanddünen. Diese Tiere erreichen eine Gesamtlänge von ca. 60 cm. Die türkise Grundfärbung verleiht den Riesensmaragdeidechsen ihren Namen. Die Männchen sind teilweise recht unverträglich.

Lacerta schreiberi oder die iberische Smaragdeidechse ist eine relativ große gedrungene Smaragdeidechse mit kräftigen Beinen, breitem Kopf und langem Schwanz. Sie wird nach ihrem Entdecker, einem Herrn Schreiber, benannt. Ihre Gesamtlänge beträgt 25 cm bis 40 cm, die Körperlänge maximal 12 cm bis 13,5 cm, wobei der Schwanz 1,5 bis 2 mal so lang wie der Körper ist. Die Rückenfärbung ausgewachsener Tiere ist variabel. Die Männchen sind in der Regel grasgrün oder gelbgrün mit zahlreichen kleinen und größeren schwarzen Flecken. Die Weibchen sind meist bräunlich oder graubraun mit Grüntönen und unregelmäßigen schwarzen Flecken, die oft dicht beieinander liegen und die Form von drei Längsreihen annehmen. Zusätzlich können weißliche Augenflecken auftreten. Jungtiere und Halbwüchsige sind ebenfalls braun und an den Flanken mit auffälligen, teilweise in 3 bis 5 Längsreihen angeordneten, gelblichen, schwarz umrandeten, Augenflecken versehen. Die Männchen, zum Teil auch ältere Weibchen tragen während der Paarungszeit eine leuchtend blaue Kehlregion. Der Bauch ist gelblich und in der Regel schwarz gefleckt.
Lacerta schreiberi kommt nur auf der iberischen Halbinsel in mehreren voneinander getrennten Populationen vor, vor allem in Nordspanien, Zentralspanien und Portugal. Sie lebt vor allem in fechten Gebieten des Hügel- und Berglandes bis in eine Höhe von maximal 1800 Meter. Diese Eidechse bewohnt vegetationsreiche Lebensräume, zum Beispiel überwachsene Straßenböschungen, Brombeerdickichte an Wegrändern oder Geröllhalden mit dichtem Kräuterbewuchs, häufig in der Nähe von Gebirgsbächen. Lacerta schreiberi ist eine relativ träge, sich gerne sonnende Eidechse, die bei Gefahr im Wasser untertauchen kann. Ihre Nahrung besteht aus größeren Gliedertieren, Insekten und Spinnen, aber auch Schnecken und kleineren Echsen.

Die Mauereidechse Podarcis muralis gehört zur Familie der Echten Eidechsen. Sie besitzt einen schwarzen Kopf, einen schlanken, mäßig abgeflachten Körper und einen langen Schwanz. Die Kopf-Rumpf-Länge beträgt bis 7,5cm, die Schwanzlänge beträgt das 1,5 bis 2,3-fache dieser Länge. Die Färbung ist sehr variabel und besteht auf der Oberseite aus den unterschiedlichsten Brauntönen. Die Männchen zeigen auch manchmal eine grünliche Zeichnung und am Übergang zwischen Flanken und Bauch auch einzelne blaue Schuppen. Vorherrschend sind an den Flanken dunkle Töne die zum Rücken meist mit einer dunklen Längslinie abschließen. Der Rücken ist meist heller und weist manchmal in der Mitte eine durchbrochene Längslinie auf. Bei den Männchen lösen sich diese Längszeichnungsmuster oft auch in Netzzeichnungsmuster auf. Die Bauchfärbung reicht bei den Weibchen von cremeweiß bis kupferbraun und ist fast einfarbig. Bei den Männchen kann der Bauch auch leuchtend gelb bis orange gefärbt sein und ist oft stark dunkel gepunktet bis fast ganz schwarz. Die Mauereidechse kommt von Nordostspanien bis Mittelspanien ostwärts über Mitteleuropa und Südeuropa vor und in den Balkanländern bis zur Westküste des Schwarzen Meeres. Im Norden reicht ihr Vorkommen bis Südengland.

Die so genannte Ruineneidechse Podarcis sicula hat im Norden Europas eine Kopf-Rumpf-Länge bis etwa 7 cm, im Süden bis etwa 9 cm. Die Länge des Schwanzes ist hierbei meist doppelt so lang wie die Kopf-Rumpf-Länge. Die Färbung und die jeweilige Zeichnung sind meist sehr variabel. Die Systematik der Ruinen-Eidechse wird zurzeit überarbeitet. Von den vielen beschriebenen Unterarten sollen hier nur die beiden, die mit großer Wahrscheinlichkeit bestehen bleiben, angeführt werden. Zum Einen Podarcis sicula sicula (Rafinesque, 1810), zum Anderen Podarcis sicula campestris (De Betta, 1857). Letztere hat als eigentliches Verbreitungsgebiet die Apeninnen-Halbinsel. Die Tiere der zentralen und südlichen Dalmatinischen Küste erscheinen eingeschleppt. Im Norden Dalmatiens ist die Lage noch nicht ganz geklärt. Dieses Tier bewohnt bevorzugt vegetationsreiche Örtlichkeiten, wird aber auch an Felsen und altem Gemäuer gefunden, oft sogar an Küstenfelsen in der Spritzwasserzone.

Podarcis melisellensis wird auch Karst-Eidechse genannt. Sie kommt in Dalmatien bevorzugt an alten Gemäuern vor.

Podarcis peloponnesiaca, auch Peloponnesische Mauereidechse genannt, hat eine Kopf-Rumpf-Länge bis etwas über 8 cm. Der Schwanz ist etwa doppelt so lang. Die Männchen werden etwas größer als die Weibchen. Die Grundfärbung des Rückens ist bräunlich, bei den Männchen auch grünlich. Die Weibchen sind mit vier hellen Längsstreifen sehr kontrastreich gefärbt. Die Männchen besitzen zwischen den Längsstreifen schwarze Makeln. Diese Art ist endemisch auf der Peloponnes-Halbinsel in Griechenland und bewohnt die Region bis ca. 1600 m über dem Meeresspiegel. Sie hat unterschiedliche Lebensräume wie Straßenränder, Olivenhaine oder Ruinen. Sie klettert nicht gern und hält sich meist am Boden auf.

Der Grundstoff für die erfindungsgemäßen Wirkstoffe wird bei den angeführten Waran- und Eidechsenarten auf die folgende Weise gewonnen, wobei die Anwendung für Rechtshänder gilt:
a) Das jeweilige Tier bei einer Umgebungstemperatur von 22 bis 26°C in die linke Hand nehmen und dem Waran oder der Eidechse ein oder mehrere sterile Wattestäbchen nähern und warten, bis das Tier in dieses Wattestäbchen beißt.
b) Das oder die betreffenden Wattestäbchen für ca. 2 Stunden zur Analyse in etwa 1 mL Reinstwasser legen.
c) Das Wasser unter sterilen Bedingungen verdunsten lassen.

Der erhaltene Wirkstoff wird in normalen Zellkulturflaschen in Anwesenheit von normalem DMEM/Ham's F12-Medium bei 37°C bebrütet und wächst in der Folge rasch. Hierbei handelt es sich offenbar um Mikroorganismen, die im Wesentlichen aus Eiweiß, also Prionen, bestehen, denn ihr Wachstum ist sowohl in aerober als auch in anaerober Atmosphäre zu verzeichnen. Dieses Wachstum der Mikroorganismen gewährleistet prinzipiell eine Lieferung auch größerer Mengen an Wirkstoff. Eine pharmazeutisch wirksame Menge dieses Wirkstoffs wird dann erhitzt, um eventuelle giftige Bestandteile zu zerstören, und in einer Kochsalzlösung aufbereitet. Das Erhitzen oder Erwärmen erfolgt bevorzugt bei einer Temperatur oberhalb von 35°C, bevorzugter oberhalb von 80°C und noch bevorzugter oberhalb von 100°C.

Die Wirksamkeit des erfindungsgemäßen Wirkstoffs wurde an nativem Gewebe überprüft. Es ergaben sich folgende Ergebnisse:
1) Mischkultur aus gesundem Gewebe und Gewebe aus Brustkrebs einer 23-jährigen Frau. Nach 3 Tagen war nur noch gesundes Gewebe auffindbar.
2) Mischkultur aus gesundem Gewebe und Gewebe aus Brustkrebs einer 54-jährigen Frau. Nach 3 Tagen war nur noch gesundes Gewebe auffindbar.
3) Mischkultur aus gesundem Gewebe und Gewebe aus Brustkrebs einer 60-jährigen Frau. Nach 4 Tagen war nur noch gesundes Gewebe auffindbar.
4) Mischgewebe aus gesundem Gewebe und aus Brustkrebs einer 38-jährigen Frau, wobei der Anteil an Tumorgewebe sehr hoch war. Nach 6 Tagen waren nur noch ca. 25% der ursprünglichen Tumorzellen vorhanden.
5) Kleinzelliges Bronchialkarzinom einer 53-jährigen Frau. Nach 5 Tagen war kein Tumorgewebe mehr auffindbar.
6) Mischgewebe aus gesundem Gewebe und aus einem Prostatakarzinom eines 47-jährigen Mannes. Nach 8 Tagen war kein Tumorgewebe mehr vorhanden.

Die erfindungsgemäßen pharmazeutischen Wirkstoffe, die aus den Sekreten der Tiere gewonnen wurden, sind insbesondere für unterschiedlichste Krebserkrankungen verwendbar. Die Anwendung schließt folgende Arten von Krebserkrankungen ein, ohne jedoch darauf beschränkt zu sein:
1) kleinzelliges Bronchialkarzinom
2) Mammakarzinom, auch pt - 21
3) Hodgkin-Lymphon
4) Non-Hodgkin-Lymphon
5) Astrozytome I,II und III
6) Glioblastom
7) Nierenzellkarzinom
8) Malignes Melanom
9) Colonkarzinom
10) Lebermetastasen (Chemokrebs)
11) Knochenmetastasen (Chemokrebs)
12) Knochenmarkmetastasen
13) Prostatakarzinom
14) Cervixkarzinom
15) Ovarialkarzinom
16) Adenokarzinome
17) Pankreaskarzinom

Dabei sind auch Metastasen der unterschiedlichen Krebserkrankungen beispielsweise im Knochenmark oder in der Leber mitumfasst.

## Patentansprüche

1. Pharmazeutischer Wirkstoff zur Behandlung von Tumorerkrankungen, enthaltend in einer pharmazeutisch wirksamen Menge zumindest ungereinigtes Mundsekret, gewonnen von Tieren der Gattung Varanus, Timon, Lacerta und/oder Podarcis.

2. Pharmazeutischer Wirkstoff gemäß Anspruch 1, **dadurch gekennzeichnet, dass** der pharmazeutische Wirkstoff ungereinigtes Mundsekret eines oder mehrerer Tiere enthält.

3. Pharmazeutischer Wirkstoff gemäß einem oder mehreren der vorangegangenen Ansprüche, **dadurch gekennzeichnet, dass** die Tiere der Gattung Varanus den Arten Varanus kingorum, Varanus gilleni, Varanus glauerti, Varanus acanthurus, Varanus similis, Varanus storri, Varanus tristis und/oder Varanus timorensis angehören.

4. Pharmazeutischer Wirkstoff gemäß einem oder mehreren der vorangegangenen Ansprüche, **dadurch gekennzeichnet, dass** die Tiere der Gattung Timon den Arten Timon lepida und/oder Timon pater angehören.

5. Pharmazeutischer Wirkstoff gemäß einem oder mehreren der vorangegangenen Ansprüche, **dadurch gekennzeichnet, dass** die Tiere der Gattung Lacerta den Arten Lacerta viridis, Lacerta trilineata und/oder Lacerta schreiberi angehören.

6. Pharmazeutischer Wirkstoff gemäß einem oder mehreren der vorangegangenen Ansprüche, **dadurch gekennzeichnet, dass** die Tiere der Gattung Podarcis den Arten Podarcis muralis, Podarcis sicula, Podarcis melisellensis und/oder Podarcis peloponnesiaca angehören.

7. Verwendung eines pharmazeutischen Wirkstoffs gemäß einem oder mehreren der vorangegangenen Ansprüche zur Herstellung eines Medikaments zur Behandlung von Karzinomen, insbesondere von Karzinomen, ausgewählt aus der Gruppe, bestehend aus kleinzelligem Bronchialkarzinom Mammakarzinom, Hodgkin-Lymphon, non-Hodgkin-Lymphon, Astrozytome, Glioblastom, Nierenzellkarzinom, malignes Melanom, Colonkarzinom, Lebermetastasen, Knochenmetastasen, Knochenmarkmetastasen, Prostatakarzinom, Cervixkarzinom, Ovarialkarzinom, Adenokarzinome und Pankreaskarzinom.

8. Verfahren zur Herstellung eines Wirkstoffes nach einem der Ansprüche 1 bis 6, **dadurch gekennzeichnet, dass** das Mundsekret in geringen Mengen
a) bebrütet wird;
b) eingeengt wird; und dann
c) gekocht wird und mit einer üblichen Kochsalzlösung versetzt wird.
